# EUROPÄISCHE PATENTANMELDUNG

(11) **EP 3 892 520 A1**
(43) Veröffentlichungstag der Anmeldung: **13.10.2021**
(21) Anmeldenummer: 21167119.3
(22) Anmeldetag: 07.04.2021
(51) Int. Cl.: B62B 3/14, B62B 5/06, A47K 10/42, A61L 2/26

(54) **BEHÄLTER FÜR REINIGUNGSTÜCHER MIT BEFESTIGUNGSVORRICHTUNG**

(30) Priorität: 08.04.2020 DE 202020101941 U
(71) Anmelder: Likosar, Ferdinand, 6719 Bludesch (AT); Likosar, Juliane, 6719 Bludesch (AT)
(72) Erfinder: Likosar, Ferdinand, 6719 Bludesch (AT); Likosar, Juliane, 6719 Bludesch (AT)
(74) Vertreter: Riebling, Peter

(57) **Zusammenfassung**

Behälter (1) für Reinigungstücher (2), wobei der Behälter (1) eine Befestigungsvorrichtung (8) für die Anordnung des Behälters (1) an einem Einkaufswagen (18) aufweist, wobei die Befestigungsvorrichtung (8) mindestens ein Pfandschlüssel (9) aufweist, der in ein Pfandschloss (20) eines Einkaufswagens (18) einsteckbar ist.

## Beschreibung

Die Erfindung betrifft einen Behälter für Reinigungstücher mit Befestigungsvorrichtung nach dem Oberbegriff des Schutzanspruches 1.

Unter einem Behälter für Reinigungstücher wird beispielsweise ein Behälter aus Kunststoff, Holz oder Bambus verstanden. Der Behälter kann auch als eine Folienverpackung, als Box, als ein Spender oder als eine Dose ausgebildet sein. Bevorzugt weist der Behälter eine wiederverschließbare Öffnung auf, um hieraus die Tücher einzeln zu entnehmen und eine Austrocknung der Tücher zu verhindern.

Unter dem Begriff Reinigungstücher werden beispielsweise Mikrofasertücher, Papiertücher oder vorgetränkte Einmaltücher (Feuchttücher) verstanden, welche zur Reinigung und Desinfizierung von glatten Oberflächen und Hautoberflächen geeignet sind.

Eine Desinfektion von Oberflächen ist bei einer Viruspandemie von hoher Bedeutung. Solche gefährdeten Oberflächen können beispielsweise Pakete, Bargeld, Treppengeländern oder Einkaufswägen sein. Beim Berühren der Oberflächen mit den Händen besteht eine Ansteckungsgefahr durch eine Schmierinfektionen. Die Erreger werden über die Hände auf die Schleimhäute der Nase, des Mundes oder der Augen, in den menschlichen Organismus übertragen. Eine gute Handhygiene ist daher eine wichtige Maßnahme, um sich vor einer Ansteckung mit einem Virus zu schützen.

Aus dem Stand der Technik sind bereits Desinfektionstücher oder antibakterielle Reinigungstücher bekannt, welche Bakterien, Viren und starken Schmutz auf den jeweiligen Oberflächen entfernen. Die Tücher sind entweder in eine dünnen Folienverpackung oder in einer starren Kunststoffbehälter erhältlich.

Ein Nachteil bei den bekannten Behältern für Reinigungstücher ist, dass diese sehr groß sind und daher nicht in eine gewöhnliche Hosen- oder Jackentasche passen. Die Behälter müssen somit stets in einer separaten Tasche mitgenommen werden. Hieraus ergibt sich jedoch der weitere Nachteil, dass die Tücher in der Tasche nicht griffbereit sind, denn es muss jedes Mal die Tasche geöffnet werden, um den Behälter herauszuholen und anschließend ein antibakterielles Tuch zu entnehmen.

Bei einem Einkauf in einem Supermarkt befinden sich die Einkaufswagen üblicherweise an einer Sammelstelle, wobei die einzelnen Wagen ineinandergeschoben und mit einer Sperrkette verbunden sind. Um einen Einkaufswagen auszuleihen muss die Sperrkette des Wagens gelöst werden. Hierfür muss in das Pfandschloss des Einkaufwagens eine Euro-Münze oder ein Einkaufswagenchip (Einkaufschip) eingesteckt werden. Nach dem Entfernen der Sperrkette kann der Einkaufswagen von den anderen Einkaufswagen getrennt und für den Einkauf im Supermarkt genutzt werden. Da der Einkaufswagen am Tag von mehreren unterschiedlichen Personen benutzt wird, besteht die Gefahr, dass sich insbesondere am Griff und/oder am Pfandschloss Viren befinden und es zu einer Schmierinfektion bei dem nachfolgenden Benutzer kommt, wenn er diese Flächen mit der Hand berührt. Zwar kann der Griff vor dem ersten Gebrauch mit einem Reinigungstuch desinfiziert werden, jedoch sind die Einkaufswagen zunächst ineinandergeschoben, so dass keine Möglichkeit besteht den Behälter für die Reinigungstücher in den Korb des Einkaufswagens zu legen oder irgendwo abzustellen, um ein Tuch für die Desinfektion des Griffs zu entnehmen.

Der Erfindung liegt deshalb die Aufgabe zugrunde, einen Behälter für Reinigungstücher so weiterzuentwickeln, dass er jederzeit, insbesondere bei einem Einkauf in einem Supermarkt, griffbereit ist und ein Reinigungstuch entnommen werden kann.

Zur Lösung der gestellten Aufgabe ist die Erfindung durch die technische Lehre des Anspruches 1 gekennzeichnet.

Wesentliches Merkmal der Erfindung ist, dass der Behälter eine Befestigungsvorrichtung aufweist, an welcher mindestens ein Pfandschlüssel angeordnet ist, welcher in das Pfandschloss des Einkaufswagens einsteckbar ist.

Mit der erfindungsgemäßen Ausführungsform ergibt sich der Vorteil, dass der Behälter für die Reinigungstücher vor dem eigentlichen Berühren des Einkaufswagens in das Pfandschloss gesteckt wird und dadurch mit dem Einkaufwagen verbunden ist. Durch das Einstecken des Pfandschlüssels in das Pfandschloss des Einkaufswagens wird eine Halterung für den Behälter mit Reinigungstüchern am Einkaufwagen geschaffen. Nach dem erfolgten Einstecken des Pfandschlüssels kann ein Reinigungstuch aus dem Behälter entnommen und alle wichtigen Flächen, welche während des Einkaufs berührt werden, mit dem Reinigungstuch gesäubert bzw. desinfiziert werden.

Wenn die Desinfizierung der Griffflächen abgeschlossen ist, wird der Einkaufswagen von den anderen Wagen getrennt und kann für den Einkauf genutzt werden. Es findet somit eine Desinfizierung des Einkaufswagens vor dessen eigentlichen Gebrauch statt, wobei die Reinigungstücher griffbereit sind.

Der erfindungsgemäße Pfandschlüssel ersetzt beispielsweise die 50-Cent, 1-Euro oder 2-Euro-Münze, bzw. den Einkaufswagenchip und entsperrt den Einkaufswagen. Der Pfandschlüssel ist hierbei so ausgebildet, dass er während des Ausleihvorgangs im Pfandschloss steckt und gleichzeigt als Halterung für den Behälter für die Reinigungstücher ausgebildet ist.

Der Pfandschlüssel kann beispielsweise als Universalschlüssel ausgebildet sein, wobei dessen Kopfumfang verschiedene Rundungen aufweist, welche den Radien der unterschiedlichen Mützen entsprechen. Der Pfandschlüssel ersetzt damit die Münze oder den Einkaufswagenchip (Einkaufswagenlöser) und entsperrt den Einkaufswagen ohne eine Münze oder einen Chip.

Bei einer ersten bevorzugten Ausführungsform ist die Befestigungsvorrichtung als Kette ausgebildet. Die Kette besteht aus mindestens drei beweglichen, ineinandergefügten oder mit Gelenken verbundenen Gliedern. Auf der einen Seite ist die Kette an dem Behälter angeordnet und auf der anderen Seite befindet sich der Pfandschlüssel. Durch die Kette ist der Pfandschlüssel beweglich an dem Behälter angeordnet und kann dadurch leicht in das Pfandschloss eingesteckt werden.

Bei einer zweiten bevorzugten Ausführungsform ist die Befestigungsvorrichtung als klappbarer Hebel ausgebildet, an dessen Ende sich der Pfandschlüssel befindet. Bevorzugt bilden der klappbare Hebel und der Pfandschlüssel eine Einheit aus. Dies bedeutet, dass der klappbare Hebel einen Kopfumfang aufweist, welcher einer 50cent, 1-Euro oder 2-Euro-Münze entspricht. Die Anordnung der hebelartigen Befestigungsvorrichtung erfolgt am Gehäuse des Behälters.

Bevorzugt ist die Befestigungsvorrichtung mit einem Lager am Gehäuse des Behälters angeordnet. Das Lager kann beispielsweise nur einen Freiheitsgrad aufweisen. Diese Ausführungsform hat den Vorteil, dass der Hebel relativ starr ausgebildet ist und nur noch nach oben oder unten geklappt werden kann. Der Behälter nimmt bei einem eingesteckten Pfandschlüssel in dem Pfandschloss eine fixe Position gegenüber dem Pfandschloss ein und kann nur in vertikale Richtung bewegt werden.

Es ist jedoch auch möglich, dass die Befestigungsvorrichtung an beiden Enden jeweils ein Lager aufweist, so dass der Behälter zwar gegenüber dem Pfandschloss fixiert ist, jedoch aufgrund seines Eigengewichts herunterhängt.

Bei einer weiteren bevorzugten Ausführungsform weist das Lager eine ArretierVorrichtung auf, mit welcher der Behälter bei eingesteckten Pfandschlüssel in dem Pfandschloss in seiner vertikalen Position gehalten wird. Dies hat den Vorteil, dass die wiederverschließbare Öffnung des Behälters stehts gut zugänglich ist und damit leicht ein Tuch entnommen werden kann.

Darüber hinaus kann die Befestigungsvorrichtung über ein Drehlager an dem Behälter angeordnet werden. Wenn nun der Pfandschlüssel im Pfandschloss eingesteckt ist, so lässt sich der Behälter aufgrund der drehenden Lagerung drehen, was eine Entnahme eines Tuchs erleichtert.

Bei einer weiteren bevorzugten Ausführungsform ist die Befestigungsvorrichtung als nutenförmige Aufnahme ausgebildet, in welche einzelne kugelkopfähnliche Verbinder ein- und ausklinkbar sind. Der Pfandschlüssel weist auf zumindest einer Seite einen Verbinder auf, welcher in die nutenförmige Aufnahme einsteckbar ist. Dadurch lassen sich unterschiedliche Pfandschlüssel mit dem Behälter verbinden.

Die Anordnung der Befestigungsvorrichtung am Gehäuse des Behälters kann durch unterschiedliche (lösbare oder nicht lösbare) Verbindungstechniken erfolgen. Beispielhaft werden an dieser Stelle einer Verbindungstechniken genannt: eine Scharnierverbindung (z.B. Filmscharnier), eine Lagerverbindung oder eine Kugel-Schnappverschluss-Verbindung.

Bei einer weiteren bevorzugten Ausführungsform weist der Behälter in seinem Gehäuse eine Vertiefung für die Befestigungsvorrichtung auf. Ist beispielsweise die Befestigungsvorrichtung als schwenkbarer Hebel ausgebildet, so kann der Hebel in die Vertiefung eingeklappt werden und steht bevorzugt nicht über den Gehäuseumfang des Behälters hinaus. Wenn die Befestigungsvorrichtung beispielsweise als Kette ausgebildet ist, so kann die Kette in die Vertiefung eingesetzt werden, wobei gleiche elastische Vorsprünge die Kette innerhalb der Vertiefung halten.

Die Befestigungsvorrichtung und der Pfandschlüssel können ferner als eine Art gleitender Schieber ausgebildet sein, welche aus dem Gehäuse des Behälters ein- und ausschiebbar ist Bei Bedarf kann der Pfandschlüssel aus dem Gehäuse geschoben werden und in das Pfandschloss eingesteckt werden. Der Schieber wird bevorzugt durch einen Schlitz im Gehäuse des Behälters geführt. Am Außenumfang des Gehäuses befindet sich ein Betätigungselement, mit welchem der Pfandschlüssel betätigbar ist.

Bei den Pfandschlösser unterscheidet man zwischen den älteren Modellen, bei denen eine Münze in einen Schieber gesteckt wird, welcher anschließend verschoben wird, um das Pfandschloss zu entsperren. Und den neueren Modellen, bei denen durch das direkte Einschieben der Münze in die Stecköffnung das Pfandschloss (Einhandbedienung) entsperrt wird.

Da bei den älteren Modellen der Pfandschlüssel nicht passt, kann der erfindungsgemäße Behälter neben der Befestigungsvorrichtung und dem Pfandschlüssel noch zusätzlich eine Aufnahme für einen Einkaufschips in der Form eines Einschubs im Boden des Behälters aufweisen.

Der Behälter für das mindestens eine Reinigungstuch ist beispielsweise als Feuchttuch-Spender ausgebildet. Bevorzugt weist der Behälter eine zylindrische Form auf und ist als Zupfdose ausgebildet, welche einen lösbaren Deckel oder einen lösbaren Boden aufweist. Die Reinigungstücher befinden sich im Innenraum der Dose.

Die Tücher sind fusselfrei und lösemittelbeständig und weisen eine antibakterielle Wirkung auf. Bevorzugt weisen die Reinigungstücher eine antibakterielle Reinigungsflüssigkeit auf. Die Tücher können entweder einzeln oder mit einem Nachfüllpack (Einlegepack) nachgefüllt werden. Bevorzugt sind circa 30 Tücher in einem Nachfüllpack vorhanden.

Nach dem Einkauf wird der Einkaufswagen wieder zu der vorgesehenen Sammelstelle des Supermarktes gebracht. Durch das Einstecken der Sperrkette wird der Pfandschlüssel freigegeben und der Behälter mit den Reinigungstüchern kann wieder mitgenommen werden. Dadurch kann der Behälter mehrfach verwendet werden.

Der Behälter ist beispielsweise käuflich in einem Supermarkt erhältlich und kann mit dem Namen des Supermarktes bedruckt sein. Es ist jedoch auch möglich, dass der Behälter mit der Marke der Reinigungstücher bedruckt ist, um eine langfristige Kundenbindung zu erreichen.

Der Erfindungsgegenstand der vorliegenden Erfindung ergibt sich nicht nur aus dem Gegenstand der einzelnen Schutzansprüche, sondern auch aus der Kombination der einzelnen Schutzansprüche untereinander.

Im Folgenden wird die Erfindung anhand von lediglich einen Ausführungsweg darstellenden Zeichnungen näher erläutert.

Es zeigen:
- Figur 1:: schematische Darstellung eines Behälters mit Reinigungstüchern
- Figur 2:: Darstellung eines zylindrischen Behälters in der Seitenansicht
- Figur 3:: Darstellung eines zylindrischen Behälters in der Seitenansicht
- Figur 4:: schematische Darstellung eines Behälters mit klappbarer Befestigungsvorrichtung und Pfandschlüssel
- Figur 5:: schematische Darstellung eines Behälters mit klappbarer Befestigungsvorrichtung und Pfandschlüssel
- Figur 6:: schematische Darstellung eines Behälters mit klappbarer Befestigungsvorrichtung und Pfandschlüssel
- Figur 7:: Draufsicht auf den Deckel eines Behälters
- Figur 8:: Seitenansicht eines Behälters mit klappbarer Befestigungsvorrichtung, Pfandschlüssel und Einschub
- Figur 9:: weitere Seitenansicht eines Behälters mit klappbarer Befestigungsvorrichtung, Pfandschlüssel und Einschub
- Figur 10:: schematische Darstellung eines Behälters mit Befestigungsvorrichtung und unterschiedlichen Pfandschlüsseln
- Figur 11:: schematische Darstellung von unterschiedlichen Pfandschlüsseln
- Figur 12:: schematische Darstellung eines Behälters mit einer Befestigungsvorrichtung und einem Chiphalter
- Figur 13:: Seitenansicht eines Behälters mit einer Befestigungsvorrichtung und einem Chiphalter
- Figur 14:: Darstellung des Bodens des Behälters mit Einschub
- Figur 15:: schematische Darstellung des Einsteckvorgangs des Behälters in das Pfandschloss eines Einkaufswagens
- Figur 16:: schematische Darstellung des Behälters eingesteckt in das Pfandschloss eines Einkaufswagens

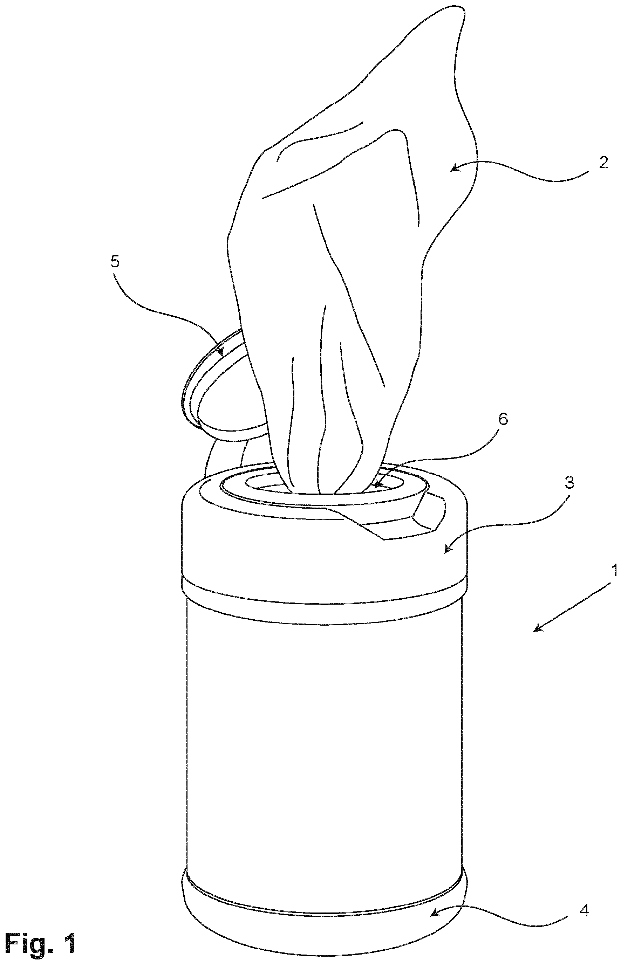

Die Figur 1 zeigt einen Behälter 1 mit einem Reinigungstuch 2. Der Behälter 1 besteht aus einem zylindrischen Grundkörper mit einem Deckel 3 und einem Boden 4. Der Deckel 3 und/oder der Boden 4 sind über einen Schraubverschluss mit dem Grundkörper des Behälters 1 verbunden.

In dem Behälter 1 befinden sich mehrere Reinigungstücher 2, welche durch eine Öffnung 6 im Deckel 3 einzeln entnommen werden können. Der Verschluss 5 ist mit einem Filmscharnier 7 mit dem Deckel 3 verbunden. Die Öffnung 6 ist mit dem klappbaren Verschluss 5 verschließbar, so dass die antibakterielle Flüssigkeit auf den Reinigungstüchern 2 nicht austrocknet.

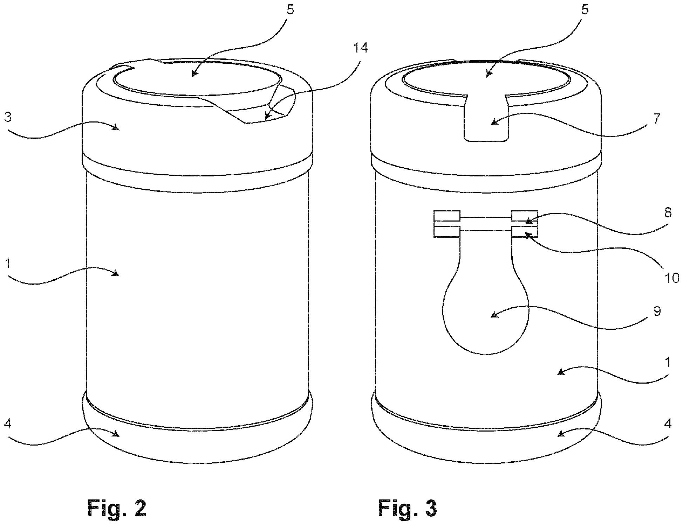

Figur 2 zeigt den Behälter 1 in einer Seitenansicht. Der Deckel 3 weist im Bereich des Verschlusses 5 einen Eingriff 14 auf. Durch den Eingriff 14 kann der Verschluss 5 leicht mit dem Finger geöffnet werden.

Mit der Figur 3 wird der Behälter 1 für Reinigungstücher 2 mit einer Befestigungsvorrichtung 8 und einem Pfandschlüssel 9 gezeigt. Die Befestigungsvorrichtung 8 besteht aus einem Lager 10, welches den Pfandschlüssel 9 beweglich aufnimmt. Der Pfandschlüssel 9 ist durch das Lager 10 klappbar am Außenumfang des Behälters 1 angeordnet.

Gemäß der Figur 3 ist der Pfandschlüssel 9 als Steckschlüssel ausgebildet, welcher in das Pfandschloss 20 eines Einkaufswagens 18 einsteckbar ist. Der Kopf des Pfandschlüssels 9 weist bevorzugt die Form einer 50cent, 1-Euro oder 2-Euro Münze auf.

Mit der Befestigungsvorrichtung 8 entsteht eine Verbindung zwischen dem Behälter 1 und dem Pfandschlüssel 9. Durch das Einstecken des Pfandschlüssels 9 in das Pfandschloss 20 des Einkaufswagens 18 wird eine Halterung für den Behälter 1 mit Reinigungstüchern 2 am Einkaufwagen 18 geschaffen.

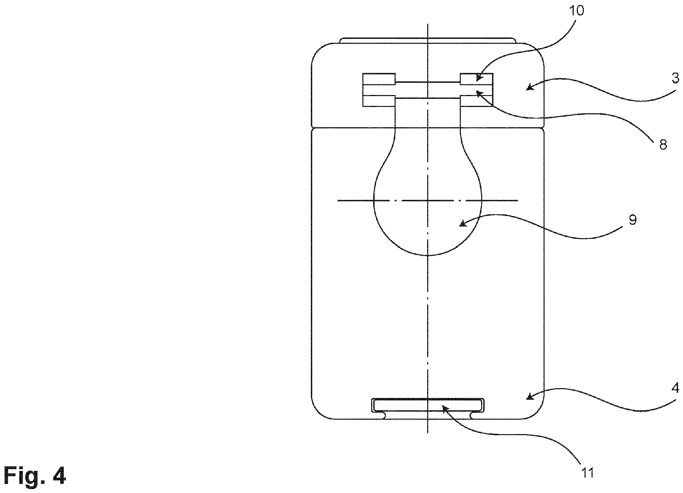

Figur 4 zeigt den Behälter 1 in einer Seitenansicht. Teilweise werden noch Pfandschlösser der 1.Generation eingesetzt, bei denen eine Münze in einen Schieber gesteckt und anschließend dieser Schieber verschoben werden muss, um das Pfandschloss zu öffnen und die Sperrkette freizugeben. Bei dieser Ausführungsform ist es nicht möglich das Pfandschloss 20 mit dem steckbaren Pfandschlüssel 9 zu entriegeln. Aus diesem Grund weist der erfindungsgemäße Behälter 1 im Boden 4 einen Einschub 11 auf, welche dazu geeignet ist einen Einkaufschip 13 oder eine Münze aufzunehmen. Der Einkaufschip 13 besteht bevorzugt aus Kunststoff und weist die Größe einer 1- oder 2-Euro-Münze auf.

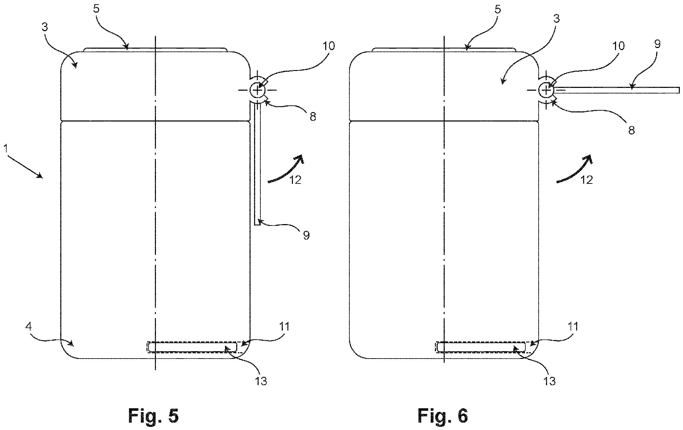

Die Figuren 5 und 6 zeigen den Behälter 1, an welchem eine klappbare Befestigungsvorrichtung 8 mit einem Pfandschlüssel 9 angeordnet ist. Die Befestigungsvorrichtung 8 besteht im Wesentlichen aus einem Lager 10, welches sich am Außenumfang des Behälters 1 befindet. Bei dem Lager 10 handelt es sich um zwei beabstandete Aufnahmen, in welche das nahezu zylindrische Ende des Pfandschlüssels 9 einsteckbar ist. Durch das Lager 10 lässt sich der Pfandschlüssel 0 von dem Gehäuse des Behälters 1 in Pfeilrichtung 12 nach oben schwenken.
Bei der vorliegenden Ausführungsform ist die Befestigungsvorrichtung 8 so ausgebildet, dass der Pfandschlüssel 9 um 90° gegenüber der Längsachse des Behälters 1 schwenkbar ist. Dadurch ergibt sich der Vorteil, dass der Pfandschlüssel 9 und der Deckel 3 mit seiner Öffnung 6 für die Reinigungstücher 2 parallel ausgerichtet sind, so dass die Tücher aus der Öffnung 6 bei einem eingesteckten Pfandschlüssel 9 im Pfandschloss des Einkaufswagens leicht zu entnehmen sind.

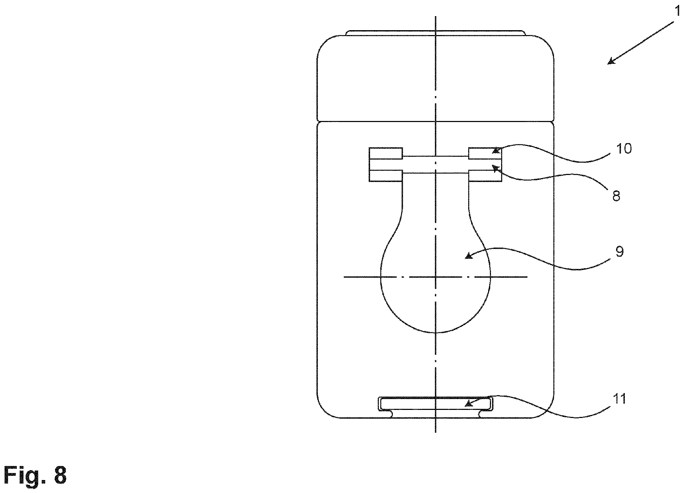

Die Figuren 5 und 8 zeigen ferner, dass sich im Boden 4 des Behälters 1 ein Einschub 11 befindet, welcher einen Einkaufschip 13 aufnimmt. Der Einschub 11 dient somit als Halterung für den Einkaufschip 13.

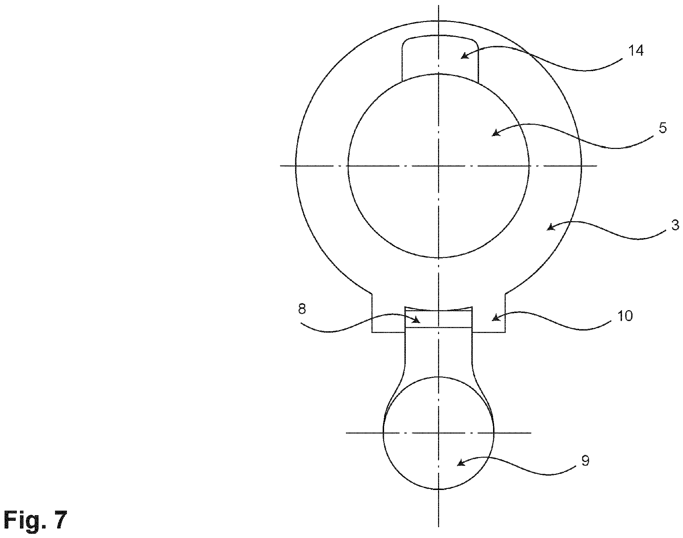

Figur 7 zeigt eine Draufsicht auf den Deckel 3 mit dem Verschluss 5 und dem Eingriff 14. Bei dieser Ausführungsform befindet sich die Befestigungsvorrichtung 8 für den Pfandschlüssel 9 am Deckel 3. Die Befestigungsvorrichtung 8 ist als Lager 8 ausgebildet, in welches der Pfandschlüssel 9 einsteckbar ist. Durch das Lager 8 kann der Pfandschlüssel 9 von dem Deckel 3 weggeklappt werden.

Mit der Figur 8 wird der Behälter 1 in einer Seitenansicht dargestellt, wobei sich am Boden 4 ein Einschub 11 für einen Einkaufschip 13 oder eine Euro-Münze befindet. Die Befestigungsvorrichtung 8 besteht aus dem Lager 10, dessen Aufnahmen am Außenumfang des Behälterkörpers angeordnet sind. In die Aufnahmen des Lagers 10 ist der Pfandschlüssel 9 einsteckbar. Der Pfandschlüssel 9 ist schwenkbar gegenüber dem Behälter 1 gelagert und kann damit leicht in das Pfandschloss 20 eines Einkaufswagens 18 gesteckt werden. Gleichzeitig kann der Pfandschlüssel 9 auch wieder an den Behälter 1 geklappt werden, wodurch der Behälter 1 platzsparend transportierbar ist.

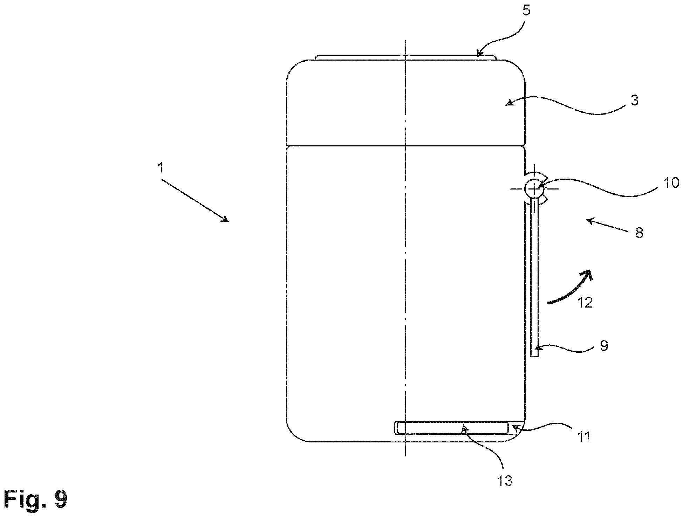

Figur 9 zeigt den Behälter 1 mit geklappten Pfandschlüssel 9. Im Bereich des Bodens 4 befindet sich der Einschub 11 für den Einkaufswagenchip 13 bzw. die Cent- oder Euro-Münze. Die Befestigungsvorrichtung 8 ist am Außenumfang des Behälters 1 angeordnet.

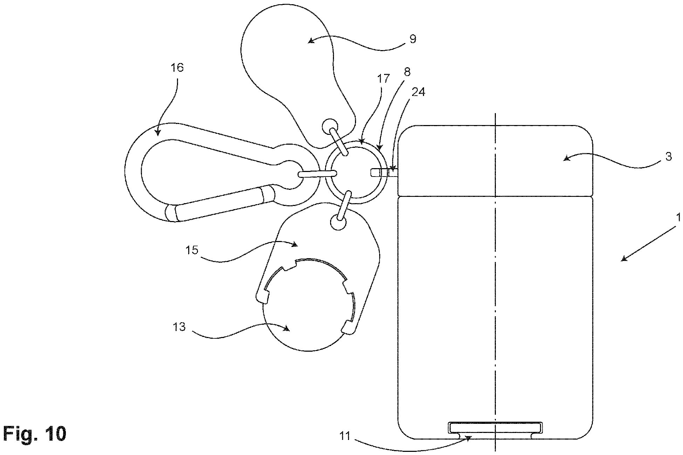

Die Figuren 10 bis 13 zeigen eine weitere Ausgestaltung der Befestigungsvorrichtung 8. Gemäß der Figur 10 ist die Befestigungsvorrichtung 8 als Ringhalter 24 ausgebildet, welcher mindestens einen Ring 17 aufnimmt. An dem Ring 17 befindet sich beispielsweise der Pfandschlüssel 9, ein Karabiner 16 oder ein Chiphalter 15 mit einem lösbaren Einkaufschip 13.

Bevorzugt lässt sich der Ringhalter 24 der Befestigungsvorrichtung 8 öffnen, so dass individuell die einzelnen Ringe 17 mit den unterschiedlichen Pfandschlüsseln 9 am Behälter 1 befestigt werden können.

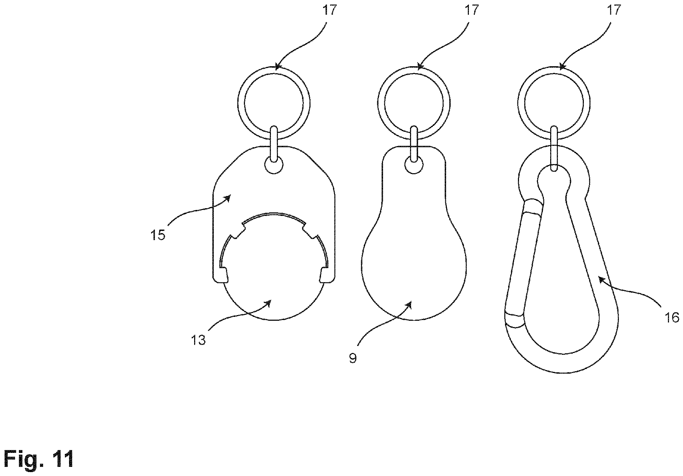

Figur 11 zeigt beispielhaft, welche unterschiedlichen Teile mit der Befestigungsvorrichtung 8 am Behälter 1 angeordnet werden können. Hierbei handelt es sich um eine Chiphalter 15 mit einem lösbaren Chip 13, einem Pfandschlüssel 9, sowie einem Karabiner 16, wobei alle Teile einen Ring 17 zur Befestigung aufweisen

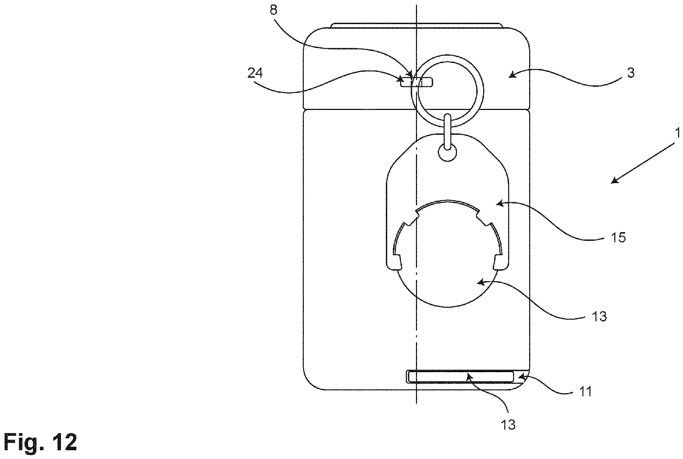

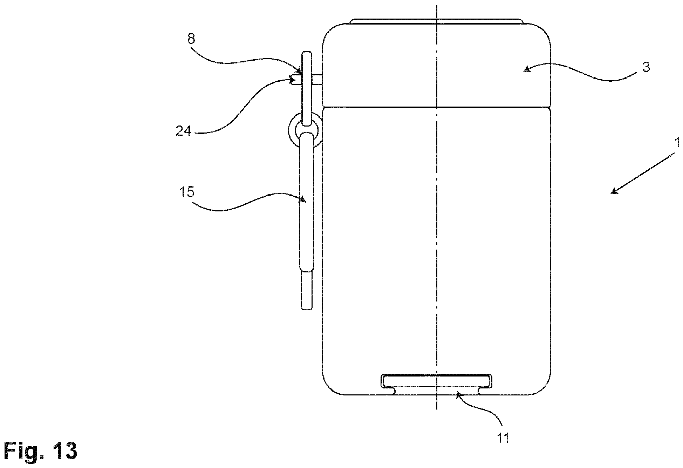

Die Figuren 12 und 13 zeigen den Behälter 1, an dessen Seitenfläche mit der Befestigungsvorrichtung 8 ein Chiphalter 15 befestigt ist. Die Befestigungsvorrichtung 8 besteht aus einem behälterseitigen Ringhalter, welcher den Ring 17 des Chiphalters 17 hält.

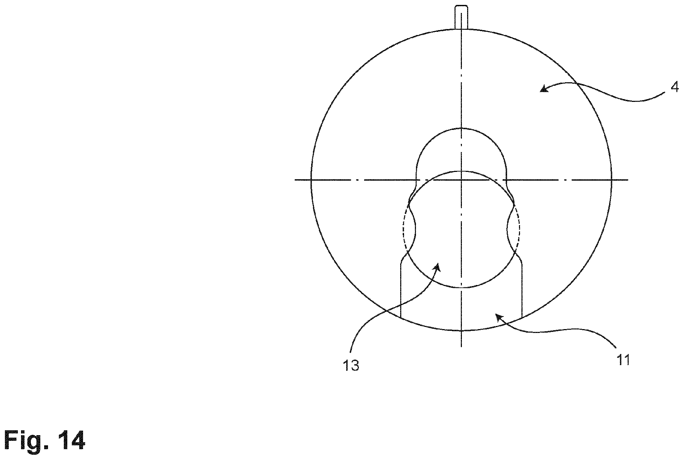

Mit der Figur 14 wird der Boden 4 des Behälters 1 gezeigt. In dem Boden 4 befindet sich ein Einschub 11 für eine Chip 13 oder eine Münze. Der Einschub 11 weist eine elastische Verjüngung auf, durch welche die Münze oder der Chip 13 innerhalb des Einschubs 11 gehalten wird.

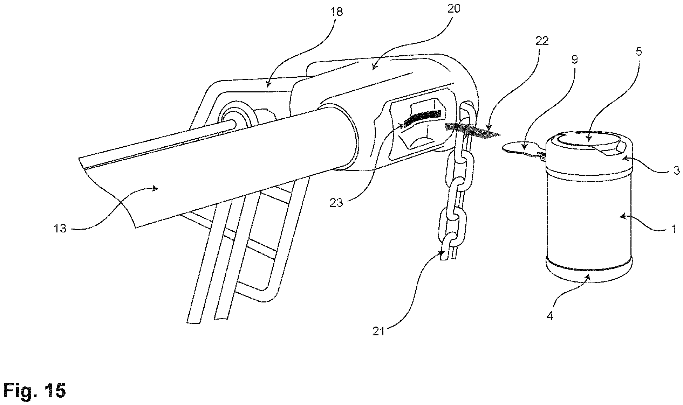

Die Figur 15 zeigt einen Einkaufswagen 18 mit einem Griff 19, an welchem ein Pfandschloss 20 angeordnet ist. Das Pfandschloss 20 weist eine Stecköffnung 23 für eine Münze oder einen Chip 13 auf. Sobald eine passende Münze oder ein Chip 12 in der Stecköffnung 23 eingeschoben wird, gibt das Pfandschloss 20 die Sperrkette 21 frei und der Einkaufswagen 18 kann von den anderen Einkaufswägen 18 getrennt werden.

Der Behälter 1 beinhaltet Reinigungstücher 2 und wird mithilfe der Befestigungsvorrichtung 8 an dem Einkaufswagen 18 befestigt. Durch die Befestigungsvorrichtung 8 ist der Pfandschlüssel 9 am Behälter 1 angeordnet, wobei der Pfandschlüssel 9 dergestalt ausgebildet ist, dass er in die Stecköffnung 23 des Pfandschlosses 20 passt und damit das Schloss auslöst und die Sperrkette 21 freigegeben wird. Der Pfandschlüssel 9 wird in Pfeilrichtung 22 in die Stecköffnung 23 eingesteckt und verbleibt während des gesamten Ausleihvorgangs des Einkaufswagens 18 in der Stecköffnung 23.

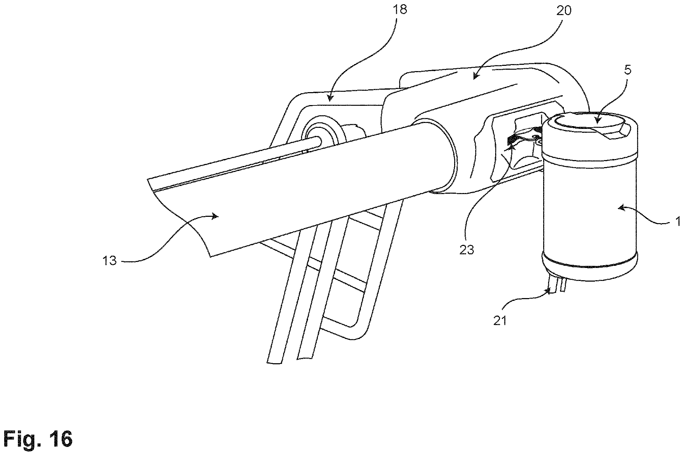

Mit der Figur 16 wird der Einkaufswagen 18 mit dem Griff 19 und dem Pfandschloss 20 gezeigt, wobei der Pfandschlüssel 9 in der Stecköffnung 23 des Pfandschlosses 20 steckt und damit der Behälter 1 am Einkaufswagen 18 angeordnet ist.

Aus der Figur 16 ergibt sich insbesondere, dass durch die Befestigungsvorrichtung 8 der Behälter 1 dergestalt am Griff 19 des Einkaufswagens 18 angeordnet ist, dass der Verschluss 5 leicht zu bedienen ist und damit die in dem Behälter 1 befindlichen Reinigungstücher 2 stets griffbereit zur Verfügung stehen.

Zusammenfassend ist zu sagen, dass mit der Befestigungsvorrichtung 8 eine gute, einfache und praktische Befestigung des Behälters 1 an dem Einkaufswagen 18 erreicht wird und damit die Reinigungstücher 2 jederzeit während des Einkaufs griffbereit sind. Da das Pfandschloss 20 meistens auf der rechten Seite am Einkaufswagengriff angeordnet ist, stört der Behälter 1 während der Benutzung des Einkaufswagens 18 nicht bzw. kaum.

### Zeichnungslegende

1. Behälter
2. Reinigungstuch
3. Deckel
4. Boden
5. Verschluss
6. Öffnung
7. Scharnier
8. Befestigungsvorrichtung
9. Pfandschlüssel
10. Lager
11. Einschub
12. Pfeilrichtung (oben)
13. Chip
14. Eingriff
15. Chiphalter
16. Karabiner
17. Ring
18. Einkaufwagen
19. Griff von 18
20. Pfandschloss
21. Sperrkette
22. Pfeilrichtung
23. Stecköffnung
24. Ringhalter

## Patentansprüche

1. Behälter (1) für Reinigungstücher (2), wobei der Behälter (1) eine Befestigungsvorrichtung (8) für die Anordnung des Behälters (1) an einem Einkaufswagen (18) aufweist, **dadurch gekennzeichnet, dass** die Befestigungsvorrichtung (8) mindestens ein Pfandschlüssel (9) aufweist, der in ein Pfandschloss (20) eines Einkaufswagens (18) einsteckbar ist.

2. Behälter (1) nach Anspruch 1, **dadurch gekennzeichnet, dass** die Befestigungsanordnung (8) beweglich ausgebildet ist und der Pfandschlüssel (9) zumindest schwenkbar gegenüber dem Gehäuse des Behälters (1) ist.

3. Behälter (1) nach einem der Ansprüche 1 oder 2, **dadurch gekennzeichnet, dass** die Befestigungsvorrichtung (8) als schwenkbarer Hebel ausgebildet ist und eine Einheit mit dem Pfandschlüssel (9) ausbildet.

4. Behälter (1) nach einem der Ansprüche 1 bis 3, **dadurch gekennzeichnet, dass** die Befestigungsvorrichtung (8) als schwenkbarer Hebel mit einer Arretiervorrichtung ausgebildet ist, wobei die Arretiervorrichtung den schwenkbaren Hebel in einer bestimmten Winkelposition (z.B. 90°) gegenüber dem Gehäuse des Behälters (1) hält.

5. Behälter (1) nach einem der Ansprüche 1 oder 2, **dadurch gekennzeichnet, dass** die Befestigungsvorrichtung (8) als Kette ausgebildet ist, an welcher ein Pfandschlüssel (9) angeordnet ist.

6. Behälter (1) nach einem der Ansprüche 1 oder 2, **dadurch gekennzeichnet, dass** die Befestigungsvorrichtung (8) als Ringhalter (24) ausgebildet ist, an welcher ein Ring (17) mit einem Pfandschlüssel (9) verbunden ist.

7. Behälter (1) nach einem der Ansprüche 1 bis 6, **dadurch gekennzeichnet, dass** der Pfandschlüssel (9) als Universalschlüssel ausgebildet ist, dessen Kopfumfang einer 50cent, 1-Euro oder 2-Euro-Münze entspricht.

8. Behälter (1) nach einem der Ansprüche 1 bis 6, **dadurch gekennzeichnet, dass** der Pfandschlüssel (9) als Chiphalter (15) ausgebildet, welcher einen Einkaufswagenchip (13) oder eine Münze lösbar aufnimmt.

9. Behälter (1) nach einem der Ansprüche 1 bis 8, **dadurch gekennzeichnet, dass** der Boden (4) des Behälters (1) ein Einschub (11) für eine Münze oder einen Einkaufswagenchip (13) aufweist.

10. Behälter (1) nach einem der Ansprüche 1 bis 9, **dadurch gekennzeichnet, dass** der Behälter (1) als Zupfdose ausgebildet ist, wobei der Deckel (3) eine Öffnung (6) für die Entnahme mindestens eines Reinigungstuches (2) aus dem Behälter (1) aufweist und die Öffnung durch einen Verschluss (5) mit einem Filmscharnier verschließbar ist.
